(19) Europäisches Patentamt
European Patent Office
Office européen des brevets

(11) Veröffentlichungsnummer: **0 023 287**
**B1**

(12) **EUROPÄISCHE PATENTSCHRIFT**

(45) Veröffentlichungstag der Patentschrift: **09.02.83**

(51) Int. Cl.³: **A 01 N 25/32** // C07D295/18

(21) Anmeldenummer: **80103849.8**

(22) Anmeldetag: **07.07.80**

(54) **Gegenmittel zum Schutz von Kulturpflanzen vor Schädigungen durch Herbizide.**

(30) Priorität: **26.07.79 DE 2930448**

(43) Veröffentlichungstag der Anmeldung: **04.02.81 Patentblatt 81/5**

(45) Bekanntmachung des Hinweises auf die Patenterteilung: **09.02.83 Patentblatt 83/6**

(84) Benannte Vertragsstaaten: **BE CH DE FR GB IT LI NL**

(73) Patentinhaber: **BAYER AG, Zentralbereich Patente, Marken und Lizenzen, D-5090 Leverkusen 1, Bayerwerk (DE)**

(72) Erfinder: **Riebel, Hans-Jochem, Dr., In der Beek 92, D-5600 Wuppertal-1 (DE)**
Erfinder: **Eue, Ludwig, Dr., Paul-Klee-Strasse 36, D-5090 Leverkusen-1 (DE)**
Erfinder: **Faust, Wilfried, Dr., Eifgenstrasse 16, D-5068 Odenthal-3 (DE)**

(56) Entgegenhaltungen:
**EP-A-0 006 540**
**EP-A-0 006 541**
**EP-A-0 006 542**
**EP-A-0 008 649**
**DE-A-2 828 265**
**DE-B-1 077 218**
**DE-B-1 134 079**
**DE-B-1 139 504**
**FR-A-2 133 793**
**FR-A-2 215 170**
**US-A-4 053 297**

**JOURNAL OF AGRICULTURAL AND FOOD CHEMISTRY, *Band 26, Nr. 1, 1968, Washington, D. C.,* US, G. R. STEPHENSON et al: »Structure activity relationships for S-ethyl N,N-Dipropylthiocarbamate (EPTC) Antidotes in Corn«, Seiten 137–140**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Gegenmittel zum Schutz von Kulturpflanzen vor Schädigungen durch Herbizide

Die vorliegende Erfindung betrifft die Verwendung von teilweise bekannten N,N'-disubstituierten Piperazinen als Gegenmittel zum Schutz von Kulturpflanzen vor Schädigungen durch herbizid wirksame Acetanilide. Ferner betrifft die vorliegende Erfindung neue Wirkstoffkombinationen, die aus teilweise bekannten N,N'-disubstituierten Piperazinen und bestimmten herbizid wirksamen Acetaniliden bestehen und besonders gute selektive herbizide Eigenschaften besitzen.

Unter »Gegenmitteln« (»Safener«, »Antidote«) sind im vorliegenden Zusammenhang Stoffe zu verstehen, welche befähigt sind, schädigende Wirkungen von Herbiziden auf Kulturpflanzen spezifisch zu antagonisieren, d. h. die Kulturpflanzen zu schützen, ohne dabei die Herbizidwirkung auf die zu bekämpfenden Unkräuter merklich zu beeinflussen.

Es ist bekannt, daß bestimmte Thiolcarbamate und Acetanilide beim Einsatz zur Unkrautbekämpfung in Mais und anderen Kulturen mehr oder weniger starke Schäden an den Kulturpflanzen hervorrufen. Weiterhin ist bekannt, daß Verbindungen wie z. B. N-Dichloracetyl-2-ethyl-piperidin und N-Dichloracetyl-cis, trans-decahydrochinolin geeignet sind, um Schädigungen durch Thiolcarbamate oder Acetanilide an Kulturpflanzen zu vermindern (vgl. DE-A-2 218 097). Die Wirksamkeit dieser Stoffe als Gegenmittel ist jedoch nicht immer ganz befriedigend.

Außerdem sind zahlreiche N,N'-disubstituierte Piperazine bereits als Stoffe mit anthelmintischen Eigenschaften beschrieben worden (vgl. DE-B-1 077 218 und DE-B-1 139 504).

Schließlich ist bereits bekannt, daß bestimmte N-substituierte Morpholine als Antidots für herbizid wirksame Thiolcarbamate und Acetanilide verwendbar sind (vgl. J. Agr. Food Chem. 26, 137–140 (1978), US-A-4 053 297 und FR-A-2 215 170). So lassen sich zum Beispiel Morpholin-Derivate, in denen das Stickstoffatom einen gegebenenfalls chlorierten Acetylrest trägt, für den angegebenen Zweck einsetzen.

Es wurde jetzt gefunden, daß die teilweise bekannten N,N'-disubstituierten Piperazine der Formel

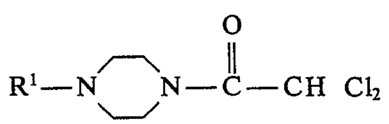

(I)

in welcher

$R^1$ für gegebenenfalls durch Halogen oder Alkoxy mit 1 bis 4 Kohlenstoffatomen substituiertes Phenyl oder für Benzyl steht,

hervorragend geeignet sind, um Kulturpflanzen vor Schädigungen durch herbizid wirksame Acetanilide der Formel

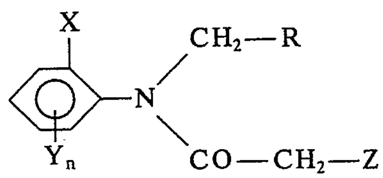

(II)

in welcher

R für einen gegebenenfalls substituierten N-haltigen heterocyclischen Rest steht,
X und Y gleich oder verschieden sind und für Alkyl stehen,
Z für Halogen steht und
n für 0, 1 oder 2 steht,

zu schützen.

Weiterhin wurde gefunden, daß die neuen Wirkstoffkombinationen bestehend aus einem N,N'-disubstituierten Piperazin der Formel (I) und mindestens einem herbizid wirksamen Acetanilid der Formel (II) hervorragend geeignet sind zur selektiven Unkrautbekämpfung in Nutzpflanzenkulturen.

Überraschenderweise werden Herbizidschädigungen durch Acetanilide der Formel (II) an Kulturpflanzen bei Mitverwendung von N,N'-disubstituierten Piperazinen der Formel (I) besser unterdrückt als beim Einsatz der bekannten Verbindungen N-Dichloracetyl-2-ethyl-piperidin und N-Dichloracetyl-cis, trans-decahydrochinolin, welches chemisch ähnliche Stoffe gleicher Wirkungsart sind. Im übrigen war nicht zu erwarten, daß die erfindungsgemäßen Wirkstoffkombinationen bessere selektive herbizide Eigenschaften besitzen als Wirkstoffkombinationen, welche aus mindestens einem

herbizid wirksamen Acetanilid der Formel (II) und dem als Gegenmittel bekannten N-Dichloracetyl-2-ethyl-piperidin oder dem ebenfalls als Gegenmittel bekannten N-Dichloracetyl-cis, trans-decahydrochinolin bestehen.

Die erfindungsgemäß verwendbaren N,N'-disubstituierten Piperazine der Formel (I) sind teilweise bekannt (vgl. DE-B-1 077 218 und DE-B-1 139 504). Die bisher noch nicht beschriebenen Stoffe der Formel (I) lassen sich in einfacher Weise nach im Prinzip bekannten Verfahren herstellen. So erhält man N,N'-disubstituierte Piperazine der Formel (I), indem man Piperazin-Derivate der Formel

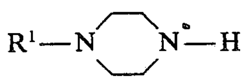

(III)

in welcher

$R^1$ die oben angegebene Bedeutung hat,

mit Dichloracetylchlorid der Formel

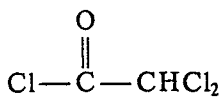

(IV)

gegebenenfalls in Gegenwart eines Verdünnungsmittels sowie gegebenenfalls in Gegenwart eines Säurebindemittels umsetzt, wobei die Verbindungen der Formel (III) auch in Form ihrer Hydrohalogenide eingesetzt werden können.

Die bei der Durchführung dieses Verfahrens als Ausgangsstoffe benötigten Piperazin-Derivate sind durch die Formel (III) allgemein definiert. In dieser Formel steht $R^1$ für diejenigen Reste, die bereits im Zusammenhang mit der Beschreibung der erfindungsgemäß verwendbaren Verbindungen für $R^1$ genannt wurden.

Die Piperazin-Derivate der Formel (III) sind bekannt oder lassen sich nach im Prinzip bekannten Verfahren herstellen. Das Dichloracetylchlorid der Formel (IV) ist ebenfalls bekannt.

Das Verfahren zur Herstellung der erfindungsgemäß verwendbaren N,N'-disubstituierten Piperazine wird vorzugsweise in Gegenwart eines Verdünnungsmittels durchgeführt. Als Verdünnungsmittel können hierbei Wasser sowie inerte organische Lösungsmittel verwendet werden. Hierzu gehören vorzugsweise Ketone, wie Diethylketon und Methylisobutylketon; Nitrile, wie Propionitril und Acetonitril; Ether, wie Tetrahydrofuran und Dioxan, aliphatische und aromatische Kohlenwasserstoffe, wie Petrolether, Benzol, Toluol oder Xylol, halogenierte Kohlenwasserstoffe, wie Methylenchlorid, Tetrachlorkohlenstoff, Chloroform oder Chlorbenzol; Ester, wie Essigester; und Formamide, wie insbesondere Dimethylformamid.

Als Säurebindemittel kommen bei der Herstellung der erfindungsgemäß verwendbaren N,N'-disubstituierten Piperazine nach dem oben beschriebenen Verfahren alle üblichen Säureakzeptoren in Betracht. Hierzu gehören vorzugsweise Alkalihydroxide, wie Natriumhydroxid und Kaliumhydroxid, ferner Alkalicarbonate, wie Natriumcarbonat, Kaliumcarbonat und Natriumhydrogencarbonat, und weiterhin niedere tertiäre Amine, wie Triethylamin, Dimethylbenzylamin, Pyridin und Diazabicyclooctan. Es kann jedoch auch im Überschuß eingesetztes Piperazin-Derivat der Formel (III) gleichzeitig als Säurebindemittel fungieren. In diesem Fall erübrigt sich die Zugabe eines zusätzlichen Säurebindemittels.

Die Reaktionstemperaturen können bei der Herstellung der erfindungsgemäß verwendbaren N,N'-disubstituierten Piperazine nach dem oben beschriebenen Verfahren in einem größeren Bereich variiert werden. Im allgemeinen arbeitet man bei Temperaturen zwischen 0°C und 100°C, vorzugsweise zwischen 10°C und 80°C.

Bei der Durchführung des oben beschriebenen Verfahrens zur Herstellung der erfindungsgemäß verwendbaren N,N'-disubstituierten Piperazine setzt man auf 1 Mol an Piperazin-Derivat der Formel (III) vorzugsweise 1 bis 2 Mol an Dichloracetylchlorid der Formel (IV) sowie gegebenenfalls 1 Mol an Säurebindemittel ein. Die Isolierung der Reaktionsprodukte erfolgt nach üblichen Methoden. Im allgemeinen verfährt man in der Weise, daß man das Reaktionsgemisch nach beendeter Umsetzung mit Wasser vermischt, das Gemisch mit einem in Wasser wenig löslichen organischen Solvens extrahiert, die vereinigten organischen Phasen abtrennt, nacheinander mit verdünnter Salzsäure und Wasser wäscht, dann trocknet, filtriert und eindampft. Der verbleibende Rückstand wird gegebenenfalls umkristallisiert, um eventuell noch anhaftende Verunreinigungen zu entfernen.

Die erfindungsgemäß verwendbaren N,N'-disubstituierten Piperazine der Formel (I) eignen sich, — wie bereits erwähnt —, zum Schutz von Kulturpflanzen vor Schädigungen durch herbizid wirksame Acetanilide der Formel (II), ohne deren Unkrautwirkung merklich zu beeinflussen.

Vorzugsweise können die N,N'-disubstituierten Piperazine als Gegenmittel zum Schutz von Kulturpflanzen vor Schädigungen durch herbizid wirksame Acetanilide der Formel (II) verwendet werden, in denen R für die gegebenenfalls substituierten Azolylreste Pyrazol-1-yl, Imidazol-1-yl, 1,2,4-Triazol-1-yl; 1,2,3-Triazol-1-yl; 1,3,4-Triazol-1-yl und 1,2,3,4-Tetrazol-1-yl sowie für gegebenenfalls substituiertes Pyrrol-1-yl steht. Als Substituenten kommen vorzugsweise in Frage: Halogen,

insbesondere Fluor, Chlor und Brom, sowie Alkyl mit 1 bis 4 Kohlenstoffatomen. X und Y sind gleich oder verschieden und stehen vorzugsweise für geradkettiges oder verzweigtes Alkyl mit 1 bis 4 Kohlenstoffatomen. Z steht vorzugsweise für die Halogene Chlor und Brom und der Index n steht für 0, 1 oder 2.

Als Beispiele für Acetanilide der Formel (II) seien im einzelnen genannt:

2-Methyl-6-ethyl-N-(pyrazol-1-yl-methyl)-chloracetanilid
2,6-Diethyl-N-(pyrazol-1-yl-methyl)-chloracetanilid
2,6-Diethyl-N-(1,2,4-triazol-1-yl-methyl)-chloracetanilid
2,6-Dimethyl-N-(1,2,4-triazol-1-yl-methyl)-chloracetanilid
2-Methyl-N-(pyrazol-1-yl-methyl)-chloracetanilid
2,5-Dimethyl-N-(pyrazol-1-yl-methyl)-chloracetanilid
2,3-Dimethyl-N-(pyrazol-1-yl-methyl)-chloracetanilid
2,6-Diethyl-N[(3,5-dimethyl-pyrazol-1-yl)-methyl]-chloracetanilid
2,6-Diethyl-N-[(3-chlor-1,2,4-triazolyl)-methyl]-chloracetanilid
2-Methyl-6-ethyl-N-[(3,5-dimethyl-pyrazol-1-yl)-methyl]-chloracetanilid
2-tert.-Butyl-N-(pyrazol-1-yl-methyl)-chloracetanilid
2-Methyl-6-ethyl-N-[(3-brom-5-methyl-pyrazol-1-yl)-methyl]-chloracetanilid
2-Methyl-6-ethyl-N-[-(3-chlor-1,2,4-triazolyl)-methyl]-chloracetanilid
2,6-Diethyl-N-[(4-chlorpyrazol-1-yl)-methyl]-chloracetanilid.

Weitere bevorzugt in Frage kommende Acetanilide der Formel (II) sind in den Herstellungsbeispielen aufgeführt.

Die Acetanilide der Formel (II) und deren herbizide Wirksamkeit sind bereits bekannt (vgl. DE-A-2 648 008 und DE-A-2 704 281).

Die erfindungsgemäß verwendbaren N,N'-disubstituierten Piperazine der Formel (I) eignen sich insbesondere zum Schutz von wichtigen Kulturpflanzen, wie Mais, Sojabohnen, Baumwolle, Zuckerrüben, Getreide, Reis und Zuckerrohr vor Herbidzidschädigungen durch Acetanilide der Formel (II).

Die erfindungsgemäßen Wirkstoffkombinationen bestehend aus einem N,N'-disubstituierten Piperazin der Formel (I) und mindestens einem herbizid wirksamen Acetanilid der Formel (II) zeigen eine sehr gute Wirkung gegen Unkräuter und Ungräser in zahlreichen Nutzpflanzenkulturen. Sie können daher zur selektiven Unkrautbekämpfung in zahlreichen Nutzpflanzenkulturen verwendet werden. — Unter Unkräutern im weitesten Sinne sind hierbei alle Pflanzen zu verstehen, die an Orten wachsen, wo sie unerwünscht sind.

Die erfindungsgemäßen Wirkstoffkombinationen können zum Beispiel bei folgenden Pflanzen angewendet werden.

Dikotyle Unkräuter der Gattungen:
Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Rorippa, Rotala, Lindernia, Lamium, Veronica, Abutilon, Emex, Datura, viola, Galeopsis, Papaver, Centaurea.
Dicotyle Kulturen der Gattungen:
Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen:
Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleotharis, Scirpus, Paspalum, Ischaemum, Spheniclea, Dactyloctenium, Agrostis, Alopecurus, Apera.
Monokotyle Kulturen der Gattungen:
Oryza, Zea, Triticum, Hordeum, Avena, Secale, Sorghum, Panicum, Saccharum, Ananas, Asparagus, Allium.

Insbesondere eignen sich die erfindungsgemäßen Wirkstoffkombinationen zur selektiven Unkrautbekämpfung in Mais, Sojabohnen, Baumwolle, Zuckerrüben, Getreide, Reis und Zuckerrohr.

Die erfindungsgemäß verwendbaren Gegenmittel können gegebenenfalls im Gemisch mit den herbiziden Wirkstoffen, bei denen sie eingesetzt werden, in die üblichen Formulierungen übergeführt werden, wie Lösungen, Emulsionen, Spritzpulver, Suspensionen, Pulver, Stäubemittel, Schäume, Pasten, lösliche Pulver, Granulate, Suspensions-Emulsionskonzentrate, wirkstoffimprägnierte Natur- und synthetische Stoffe, Feinstverkapselungen in polymeren Stoffen und in Hüllmassen für Saatgut.

Diese Formulierungen werden in bekannter Weise hergestellt, zum Beispiel durch Vermischen der erfindungsgemäß verwendbaren Gegenmittel gegebenenfalls im Gemisch mit den herbiziden Wirkstoffen, bei denen sie eingesetzt werden, mit Streckmitteln, also flüssigen Lösungsmitteln und/oder festen Trägerstoffen, gegebenenfalls unter Verwendung von oberflächenaktiven Mitteln,

also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon, oder Cyclohexanon, stark polare Lösungsmittel, wie Dimethylformamid und Dimethylsulfoxid sowie Wasser; natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel; nichtionogene und anionische Emulgatoren, wie Polyoxyethylen-Fettsäure-Ester, Polyoxy-ethylen-fettalkohol-Ether, z. B. Alkylaryl-polyglykol-ether, Alkylsulfonate, Alkylsulfate, Arylsulfonate sowie Eiweißhydrolysate; als Dispergiermittel: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% an Gegenmittel bzw. an Gegenmittel und herbizidem Wirkstoff, vorzugsweise zwischen 0,5 und 90%.

Die erfindungsgemäß verwendbaren Gegenmittel können als solche oder in ihren Formulierungen auch in Mischung mit herbiziden Wirkstoffen eingesetzt werden, wobei Fertigformulierung oder Tankmischung möglich ist. Auch eine Mischung mit anderen bekannten Wirkstoffen wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die erfindungsgemäß verwendbaren Gegenmittel bzw. Gemische aus erfindungsgemäß verwendbaren Gegenmitteln und herbizidem Wirkstoff können als solche, in Form ihrer Formulierungen oder den daraus durch weiteres Verdünnen bereiteten Anwendungsformen, wie gebrauchsfertige Lösungen, Suspensionen, Emulsionen, Pulver und Granulate angewandt werden. Die Anwendung geschieht in üblicher Weise, z. B. durch Gießen, Spritzen, Sprühen, Stäuben oder Streuen, Trockenbeizen, Feuchtbeizen, Naßbeizen, Schlämmbeizen oder Inkrustrieren.

Die erfindungsgemäßen Gegenmittel können nach den für derartige Antidote üblichen Methoden ausgebracht werden. So können die erfindungsgemäßen Gegenmittel entweder vor oder nach dem Herbizid ausgebracht oder zusammen mit dem Herbizid appliziert werden. Ferner können Kulturpflanzen durch Saatgutbehandlung mit den Gegenmitteln vor der Saat (Beizung) vor Schäden geschützt werden, wenn das Herbizid vor oder nach der Saat angewendet wird. Eine weitere Einsatzmöglichkeit besteht darin, daß man die Gegenmittel bei der Aussaat in die Saatfurche ausbringt. Wenn es sich bei den Pflanzen um Stecklinge handelt, so können diese vor. der Auspflanzung mit den Gegenmitteln behandelt werden.

Beim Einsatz der erfindungsgemäßen Gegenmittel kommen die ortsüblichen Aufwandmengen an den jeweiligen Herbiziden zur Anwendung. Die Aufwandmengen an herbizidem Wirkstoff schwanken zwischen 0,5 und 5 kg/ha. Die Aufwandmenge an Gegenmittel ist unabhängig vom Herbizid und der Aufwandmenge des herbiziden Wirkstoffes. Im allgemeinen liegen die Aufwandmengen an erfindungsgemäßen Gegenmitteln bei Flächenbehandlung zwischen 0,1 und 5 kg/ha, vorzugsweise zwischen 0,2 und 4 kg/ha. Bei der Saatgutbehandlung liegen die Aufwandmengen an erfindungsgemäßen Gegenmitteln im allgemeinen zwischen 10 und 300 g pro Kilogramm Saatgut, vorzugsweise zwischen 25 und 200 g pro Kilogramm Saatgut.

In den erfindungsgemäßen Wirkstoffkombinationen können die Gewichtsverhältnisse von Gegenmitteln zu herbiziden Wirkstoffen in relativ großen Bereichen schwanken. Im allgemeinen entfallen auf 1 Gewichtsteil an herbizidem Wirkstoff 0,05 bis 1,0 Gewichtsteile, vorzugsweise 0,1 bis 0,5 Gew.-Teile an Gegenmittel der Formel (I).

Die gute Wirksamkeit der erfindungsgemäßen Gegenmittel geht aus dem nachfolgenden Beispiel hervor.

## Beispiel A

### Pre-emergence-Test

Lösungsmittel: 5 Gew.-Teile Aceton
Emulgator: 1 Gew.-Teil Alkylarylpolyglykolether

Zur Herstellung einer zweckmäßigen Wirkstoffzubereitung vermischt man 1 Gew.-Teil herbiziden Wirkstoff bzw. Gegenmittel bzw. eines Gemisches aus Gegenmittel und herbizidem Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Herbizid-Zubereitung bzw. mit der Gegenmittel-Zubereitung bzw. mit der Zubereitung aus Gegenmittel und herbizidem Wirkstoff begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0% = keine Wirkung (wie unbehandelte Kontrolle)
100% = totale Vernichtung

Wirkstoffe, Aufwandmengen und Resultate gehen aus der nachfolgenden Tabelle hervor:

Tabelle A

Pre-emergence-Test

| Wirkstoff Herbizid | Wirkstoffaufwand an Herbizid kg/ha | Wirkstoff Gegenmittel | Wirkstoffaufwand an Gegenmittel kg/ha | % Schädigung bei Mais | Echinochloa | Amaranthus |
|---|---|---|---|---|---|---|
| | – | 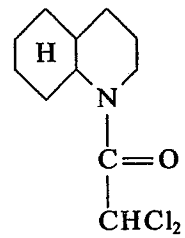 (bekannt) | 3 | 0 | 0 | 0 |
| | – | 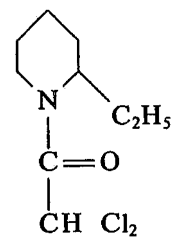 (bekannt) | 3 | 0 | 0 | 0 |
| | – | 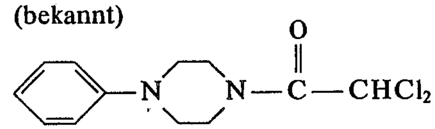 (erfindungsgemäß) | 3 | 0 | 0 | 0 |
| | – | 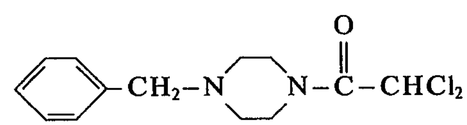 (erfindungsgemäß) | 3 | 0 | 0 | 0 |

Fortsetzung

| Wirkstoff Herbizid | Wirkstoffaufwand an Herbizid kg/ha | Wirkstoff Gegenmittel | Wirkstoffaufwand an Gegenmittel kg/ha | % Schädigung bei Mais | Echinochloa | Amaranthus |
|---|---|---|---|---|---|---|
| – | – | 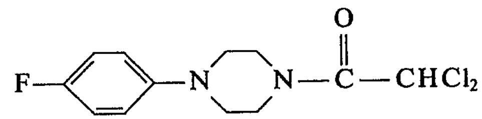 (erfindungsgemäß) | 3 | 0 | 0 | 0 |
| – | – | 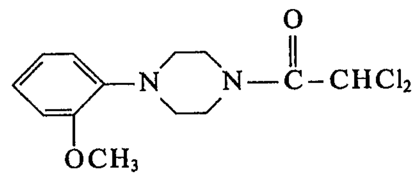 (erfindungsgemäß) | 3 | 0 | 0 | 0 |
| 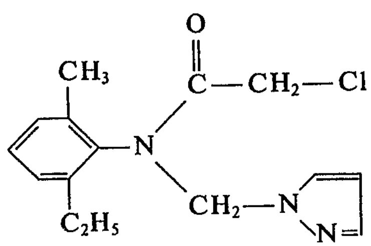 | 3 | – | – | 90 | 100 | 100 |
| 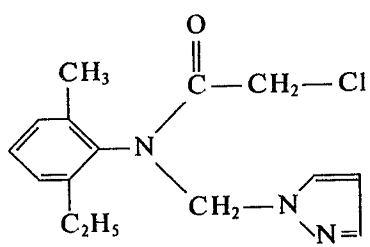 | 3 | 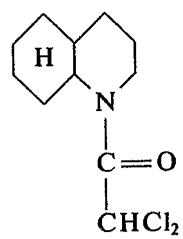 (bekannt) | 3 | 80 | 100 | 100 |

Fortsetzung

| Wirkstoff Herbizid | Wirkstoffaufwand an Herbizid kg/ha | Wirkstoff Gegenmittel | Wirkstoffaufwand an Gegenmittel kg/ha | % Schädigung bei Mais | Echinochloa | Amaranthus |
|---|---|---|---|---|---|---|
| 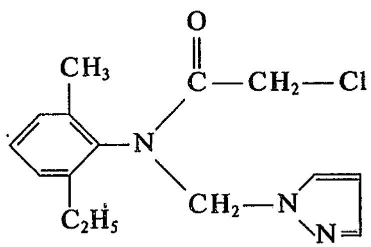 | 3 | 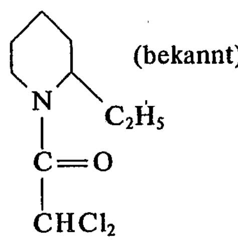 (bekannt) | 3 | 70 | 100 | 100 |
| 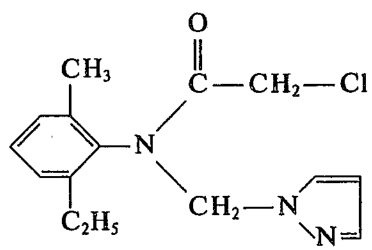 | 3 | 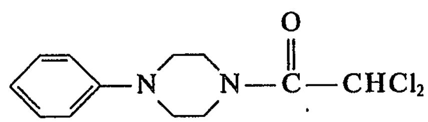 (erfindungsgemäß) | 1 | 0 | 100 | 100 |
| 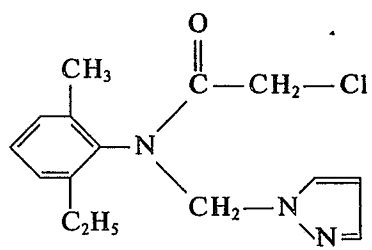 | 3 | 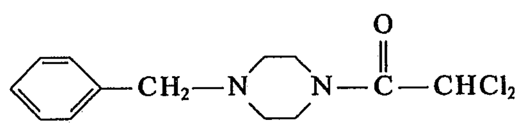 (erfindungsgemäß) | 1 | 0 | 100 | 100 |

Fortsetzung

| Wirkstoff Herbizid | Wirkstoffaufwand an Herbizid kg/ha | Wirkstoff Gegenmittel | Wirkstoffaufwand an Gegenmittel kg/ha | % Schädigung bei Mais | Echinochloa | Amaranthus |
|---|---|---|---|---|---|---|
| 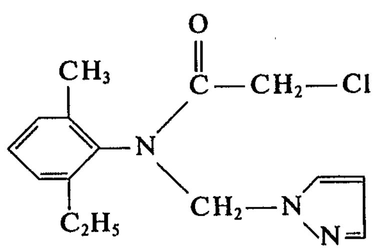 | 3 | 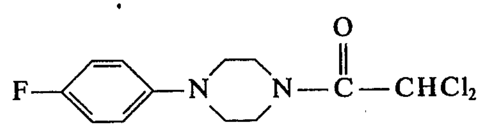 (erfindungsgemäß) | 1 | 0 | 100 | 100 |
| 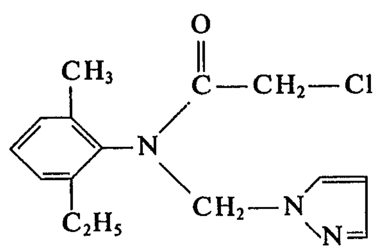 | 3 | 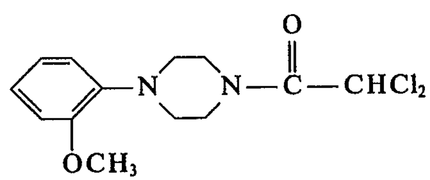 (erfindungsgemäß) | 1 | 0 | 100 | 100 |

## Herstellungsbeispiele

### Beispiel 1

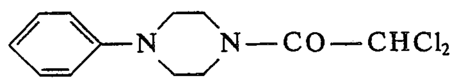

Zu einer Lösung von 8,1 g (0,05 Mol) 1-Phenyl-piperazin und 5,1 g (0,05 Mol) Triethylamin in 100 ml Toluol werden bei Raumtemperatur unter Rühren 7,4 g (0,05 Mol) Dichloracetylchlorid hinzugetropft. Es wird 3 Stunden bei 20° C nachgerührt. Danach wird aufgearbeitet, indem man das Reaktionsgemisch zweimal mit je 100 ml Wasser wäscht, die organische Phase abtrennt, trocknet und unter vermindertem Druck eindampft. Der verbleibende Rückstand wird aus Toluol umkristallisiert. Man erhält 1-(Phenyl)-4-dichloracetyl-piperazin in Form hellgelber Kristalle vom Schmelzpunkt 128° C.

Nach der im Beispiel 1 angegebenen Methode werden auch die in der nachfolgenden Tabelle 1 formelmäßig aufgeführten N,N'-disubstituierten Piperazine hergestellt.

Tabelle 1

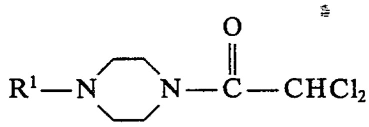

(I)

| Beispiel Nr. | $R^1$ | Schmelzpunkt (° C) |
|---|---|---|
| 2 | 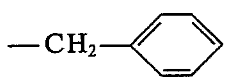 | 97 |
| 3 | 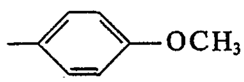 | 123 |
| 4 | 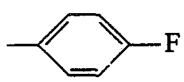 | 81 |
| 5 | 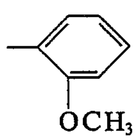 | Öl |
| 6 | 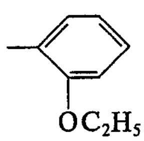 | 88 |
| 7 | 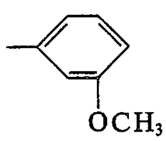 | Öl |
| 8 | 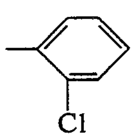 | 125 |
| 9 | 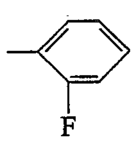 | 85 |

Die in der Tabelle 1 aufgeführten Verbindungen wurden durch ihre $^1$H-Kernresonanzspektren charakterisiert.

Beispiel (II-1)

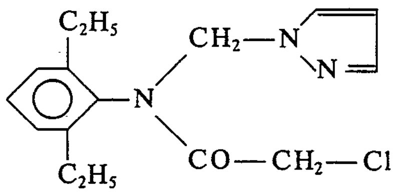

Zu 274,2 g (1 Mol) 2,6-Diethyl-N-chlormethyl-chloracetanilid in 250 ml wasserfreiem Essigester gibt man unter Rühren eine Mischung aus 68 g (1 Mol) Pyrazol und 106 g (1,05 Mol) Triethylamin in 150 ml wasserfreiem Essigester, wobei die Temperatur auf 30°C ansteigt. Man rührt 1 Stunde bei Raumtemperatur nach. Für die Aufarbeitung ergeben sich zwei Möglichkeiten:

1. Das Reaktionsgemisch wird filtriert, das Filtrat mit Wasser neutral gewaschen, über Natriumsulfat getrocknet und im Vakuum eingedampft. Nach einer fraktionierten Kristallisation mit Ligroin erhält man 171,2 g (56% der Theorie) 2,6-Diethyl-N-(pyrazol-1-yl-methyl)-chloracetanilid vom Schmelzpunkt 67° C in Form farbloser Kristalle.
2. Das Reaktionsgemisch wird auf 0°C abgekühlt, filtriert und der Filterrückstand mit 10 ml kaltem Essigester nachgewaschen. In das Filtrat werden bei 0 bis −10°C 50 g (1,4 Mol) trockener Chlorwasserstoff eingeleitet. Man saugt anschließend die ausgefallenen Hydrochlorid-Salze ab, wäscht mit 50 ml kaltem Essigester nach und verteilt den festen Rückstand zwischen 0,5 l Essigester und 0,5 l wäßriger Natriumhydroxid-Lösung mit einem pH-Wert von 12. Die organische Phase wird abgetrennt, zweimal mit je 0,5 l Natriumchloridlösung gewaschen, über Natriumsulfat getrocknet und im Vakuum eingedampft. Der farblose ölige Rückstand wird mit 60 ml Benzin versetzt, wobei er kristallisiert. Man erhält 220,2 g (72% der Theorie) 2,6-Diethyl-N-(pyrazol-1-yl-methyl)-chloracetanilid vom Schmelzpunkt 67° C in Form farbloser Kristalle.

In analoger Weise werden die in der nachfolgenden Tabelle aufgeführten Verbindungen hergestellt:

Tabelle 2

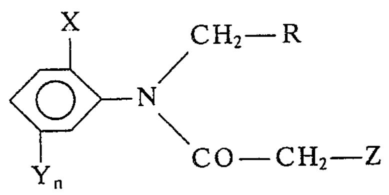

(II)

| Bsp. Nr. | X | Yn | Z | R | Schmelz-punkt (°C) |
|---|---|---|---|---|---|
| II-2 | $C_2H_5$ | 6-$C_2H_5$ | Cl | 1,2,4-Triazol-1-yl | 112 |
| II-3 | i-$C_3H_7$ | 6-i-$C_3H_7$ | Cl | Pyrazol-1-yl | 134 |
| II-4 | $CH_3$ | 6-$C_2H_5$ | Cl | 1,2,4-Triazol-1-yl | 92 |
| II-5 | $CH_3$ | 6-$C_2H_5$ | Cl | Pyrazol-1-yl | 57 |
| II-6 | $C_2H_5$ | 4,6-$(CH_3)_2$ | Cl | Pyrazol-1-yl | 32 |
| II-7 | $CH_3$ | 4,6-$(CH_3)_2$ | Cl | Pyrazol-1-yl | 92 |
| II-8 | $C_2H_5$ | 4-$CH_3$ 6-$C_2H_5$ | Cl | Pyrazol-1-yl | 78 |
| II-9 | i-$C_3H_7$ | 6-i-$C_3H_7$ | Cl | 1,3,4-Triazol-1-yl | 196 |
| II-10 | i-$C_3H_7$ | 6-i-$C_3H_7$ | Cl | 1,2,4-Triazol-1-yl | 138 |
| II-11 | $C_2H_5$ | 6-$C_2H_5$ | Cl | Pyrrol-1-yl | Oel |
| II-12 | i-$C_3H_7$ | – | Cl | 1,2,4-Triazol-1-yl | 118 |
| II-13 | $CH_3$ | 6-$C_2H_5$ | Cl | 1,2,3,4-Tetrazol-1-yl | Oel |
| II-14 | i-$C_3H_7$ | – | Cl | Pyrazol-1-yl | Oel |
| II-15 | $C_2H_5$ | – | Cl | 1,2,4-triazol-1-yl | 81 |
| II-16 | $CH_3$ | 6-$CH_3$ | Cl | Pyrazol-1-yl | 82 |
| II-17 | $CH_3$ | 6-$CH_3$ | Cl | 1,2,4-Triazol-1-yl | 110 |
| II-18 | $CH_3$ | 5-$CH_3$ | Cl | 1,2,4-Triazol-1-yl | Oel |
| II-19 | $CH_3$ | – | Cl | Pyrazol-1-yl | 56 |
| II-20 | $CH_3$ | – | Cl | 1,2,4-Triazol-1-yl | 88 |
| II-21 | $CH_3$ | 5-$CH_3$ | Cl | Pyrazol-1-yl | Oel |
| II-22 | $CH_3$ | 3-$CH_3$ | Cl | 1,2,4-Triazol-1-yl | 114 |
| II-23 | $CH_3$ | 3-$CH_3$ | Cl | Pyrazol-1-yl | 102 |
| II-24 | $C_2H_5$ | 6-$C_2H_5$ | Cl | 3,5-Dimethyl-pyrazol-1-yl | 111 |
| II-25 | $C_2H_5$ | 6-$C_2H_5$ | Cl | Brom-methyl-pyrazolyl | 145 |
| II-26 | $C_2H_5$ | 6-$C_2H_5$ | Cl | 3-Chlor-1,2,4-triazol-1-yl | 110 |

Fortsetzung

| Bsp. Nr. | X | Yn | Z | R | Schmelz-punkt (°C) |
|---|---|---|---|---|---|
| II-27 | $CH_3$ | 6-$C_2H_5$ | Cl | 3,5-Dimethyl-pyrazol-1-yl | 90 |
| II-28 | $C_2H_5$ | 6-$C_2H_5$ | Cl | 3-Methyl-pyrazol-1-yl | 89 |
| II-29 | $C_2H_5$ | 6-$CH_3$ | Cl | 3-Methyl-pyrazol-1-yl | 113 |
| II-30 | $C(CH_3)_3$ | – | Cl | Pyrazol-1-yl | Oel |
| II-31 | $C(CH_3)_3$ | – | Cl | 1,2,4-Triazolyl-1-yl | 118 |
| II-32 | $C_2H_5$ | 6-$CH_3$ | Cl | Brom-methyl-pyrazolyl | 80 |
| II-33 | $CH_3$ | 6-$C_2H_5$ | Cl | 4-Chlor-pyrazol-1-yl | 91 |
| II-34 | $CH_3$ | 6-$C_2H_5$ | Cl | 3-Chlor-1,2,4-triazol-1-yl | 121 |
| II-35 | $C_2H_5$ | 6-$CH_3$ | Cl | 2,4,5-Trichlor-imidazol-1-yl | 158 |
| II-36 | $C_2H_5$ | 6-$C_2H_5$ | Cl | 4-Chlor-pyrazol-1-yl | 110 |
| II 37 | $C_2H_5$ | 6-$C_2H_5$ | Cl | 1,2,3,4-Tetrazol-1-yl | 110 |
| II-38 | $C_2H_5$ | 6-$C_2H_5$ | Br | Pyrazol-1-yl | 68 |
| II-39 | $CH_3$ | 6-$C_2H_5$ | Br | Pyrazol-1-yl | 67 |
| II-40 | $C_2H_5$ | 6-$C_2H_5$ | Cl | Imidazol-1-yl | Oel |
| II-41 | $C_2H_5$ | 6-$C_2H_5$ | Br | 1,2,4-Triazol-1-yl | 90 |
| II-42 | $CH_3$ | 6-$C_2H_5$ | Br | 1,2,4-Triazol-1-yl | 78 |

**Patentansprüche**

1. Verwendung von N,N'-disubstituierten Piperazinen der Formel

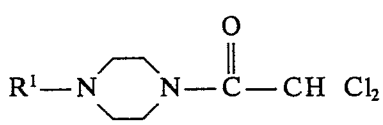

(I)

in welcher

$R^1$ für gegebenenfalls durch Halogen oder Alkoxy mit 1 bis 4 Kohlenstoffatomen substituiertes Phenyl oder für Benzyl steht,

als Gegenmittel zum Schutz von Kulturpflanzen vor Schädigung durch herbizid wirksame Acetanilide der Formel

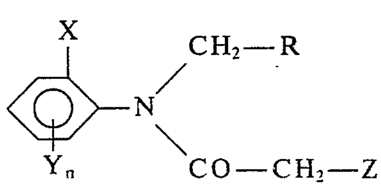

(II)

in welcher

R für einen gegebenenfalls substituierten N-haltigen heterocyclischen Rest steht,
X und Y gleich oder verschieden sind und für Alkyl stehen,
Z für Halogen steht und
n für 0, 1 oder 2 steht.

2. Mittel zur selektiven Unkrautbekämpfung in Nutzpflanzenkulturen, gekennzeichnet durch einen Gehalt an einer Wirkstoffkombination bestehend aus mindestens einem N,N'-disubstituierten Piperazin der Formel (I) gemäß Anspruch 1 und mindestens einem herbizid wirksamen Acetanilid der Formel (II).

3. Verwendung von Wirkstoffkombinationen gemäß Anspruch 2 zur selektiven Unkrautbekämpfung in Nutzpflanzenkulturen.

**Claims**

1. Use of N,N'-disubstituted piperazines of the formula

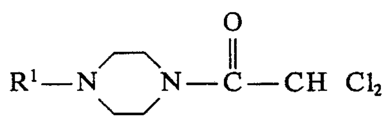
(I)

in which

$R^1$ represents phenyl which is optionally substituted by halogen or alkoxy having 1 to 4 carbon atoms, or benzyl,

as antidotes for protecting crop plants form damage by herbicidally active acetanilides of the formula

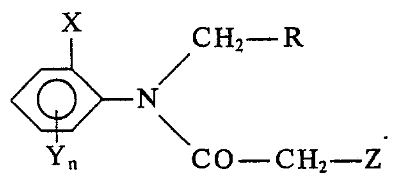
(II)

in which

R represents an N-containing heterocyclic radical which is optionally substituted,
X and Y are identical or different and represent alkyl,
Z represents halogen and
n represents 0, 1 or 2.

2. Agents for selectively combating weeds in crops of useful plants, characterised in that they contain an active compound combination consisting of at least one N,N'-disubstituted piperazine of the formula (I) according to claim 1 and at least one herbicidally active acetanilide of the formula (II).

3. Use of active compound combinations according to claim 2 for selectively combating weeds in crops of useful plants.

**Revendications**

1. Utilisation de pipérazines N,N'-disubstituées de formule:

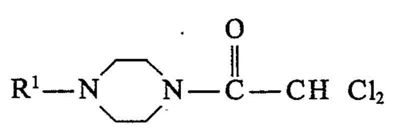
(I)

dans laquelle

$R^1$ est un groupe phényle éventuellement substitué par un halogène ou un radical alkoxy ayant 1 à 4 atomes de carbone ou le groupe benzyle,

comme antidote pour protéger des plantes cultivées contre une altération par des acétanilides à activité herbicide de formule:

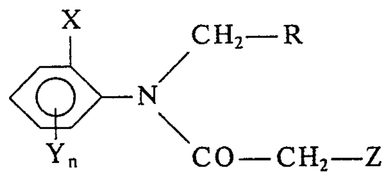

(II)

dans laquelle

R est un reste hétérocyclique contenant de l'azote éventuellement substitué,
X et Y sont égaux ou différents et représentent un groupe alkyle,
Z est un halogène, et
n est égal à 0, 1 ou 2.

2. Compositions destinées à la lutte sélective contre des mauvaises herbes dans des cultures de plantes utiles, caractérisées par une teneur en une association de substances actives formée d'au moins une pipérazine N,N'-disubstituée de formule (I) suivant la revendication 1 et d'au moins un acétanilide de formule (II) à activité herbicide.

3. Utilisation d'associations de substances actives suivant la revendication 2 dans la lutte sélective contre les mauvaises herbes dans des cultures de plantes utiles.